# EUROPEAN PATENT APPLICATION

(11) **EP 4 821 594 A1**
(43) Date of publication of application: **14.10.2026**
(21) Application number: 25169498.0
(22) Date of filing: 09.04.2025
(51) Int. Cl.: B01J 21/06, B01J 23/06, B01J 37/02, C07C 29/153

(54) **METHOD OF PRODUCING A CATALYST FOR SYNTHESIZING METHANOL FROM CARBON DIOXIDE AND HYDROGEN, THE CATALYST, AND A FLOW REACTOR AND A METHOD WITH AND A USE OF THE CATALYST**

(71) Applicant: TOYOTA JIDOSHA KABUSHIKI KAISHA, Toyota-shi, Aichi-ken, 471-8571 (JP)
(72) Inventor: NGUYEN, Phuc Hai, 1140 BRUSSELS (BE); PHONGPRUEKSATHAT, Nat, 2628 CD DELFT (NL); URAKAWA, Atsushi, 2628 CD DELFT (NL)
(74) Representative: Cabinet Beau de Loménie

(57) **Abstract**

A method of producing a catalyst for synthesizing methanol from carbon dioxide and hydrogen via a catalysis is provided, comprising a zirconium oxide support and oxide sites provided on the support including catalytic sites of zinc oxide as well as sites for capturing carbon dioxide and of an oxide of an alkaline metal and/or alkaline earth metal, the oxide sites being provided from impregnating with one or more precursor solutions for forming oxide crystals, the method comprising
providing a dry material of the zirconium oxide support,
impregnating the dry material with the one or more aqueous precursor solutions, and
removing solvent from the one or more aqueous precursor solutions,
wherein the one or more aqueous precursor solutions contain separately or together dissolved salts of zinc and of the alkaline metal and/or alkaline earth metal.

## Description

### BACKGROUND OF THE INVENTION

### 1. Field of the invention

The present disclosure relates to a method of producing a catalyst for synthesizing methanol from carbon dioxide and hydrogen via catalysis, the catalyst, a flow reactor for synthesizing methanol from carbon dioxide and hydrogen comprising the catalyst, a related method and a use of the catalyst.

### 2. Description of Related Art

The reduction of carbon dioxide using hydrogen is a key process in the production of carbon neutral fuels. The synthesis of methanol from carbon dioxide and hydrogen is such a process.

For a low pressure synthesis of methanol from carbon dioxide and hydrogen, catalysts with catalytic sites on an inorganic support are known.

JP 2024 110501 A and WO 2024/029584 A1 recommend composite oxide catalysts prepared with co-precipitation of oxides. JP 2011 83724 A shows a precipitation from a solution with salts of Ba, Na, Mg, Cu followed by depositing Pd. A catalyst obtained from wet impregnating zirconia powder with a solution of zinc nitrate is shown in Zou et al., Design of technical ZnO/ZrO2 catalysts for CO2 hydrogenation to green methanol, J. Catal. 2024, 430, 115344. This prior art as well as Nikolajsen et al., Surface ZnOx on zirconia is highly active for high temperature methanol synthesis, J. Catal. 2024, 431, 115389 recommend significantly high pressures (like 5 MPa or 50 bar) and temperatures in a fixed bed reactor with a catalyst for synthesizing methanol from carbon dioxide and hydrogen which are typically provided together in a reactor. The gas swing operation is another synthesizing approach known in chemical engineering, wherein a first and a second gas to be reacted with each other are provided to a catalyst as separate gas streams, a gas stream of the second gas replacing a gas stream of the first gas, e.g. after a purging operation with, e.g., an inert gas. The gas swing operation requires specific operating parameters, including gas concentrations, temperatures, and cycling periods. Generally, the formation and dispersion of oxides, in particular including ZnO particles over ZrO₂ is a complex task but important to provide active sites for activation of CO₂ and H₂ for reducing carbon dioxide. The active sites must be suitable for formation of a target product like methanol.

With prior art catalysts and methods with regard to aspects of synthesizing methanol from carbon dioxide and hydrogen, improvement is desired with regard to, singly or in combinations:
- efficiently making use of a carbon dioxide source of low quality and/or low concentration,
- energy efficiency,
- cost efficiency in obtaining a catalyst,
- catalyst performance, in particular at moderate pressure, in particular below 1 MPa,
- methanol productivity,
- methanol production rate,
- selectivity in synthesizing methanol from carbon dioxide and hydrogen.

### SUMMARY

It is an object of the invention to improve efficiency of the synthesis of methanol from carbon dioxide and hydrogen at moderate temperature and pressure conditions. It is an object of the invention to allow an excellent catalyst performance in the synthesis of methanol from carbon dioxide and hydrogen, in particular in terms of selectivity. It is an object of the invention to improve energy efficiency of the synthesis of methanol from carbon dioxide and hydrogen. It is an object of the invention to offer a catalyst for the synthesis of methanol from carbon dioxide and hydrogen which is obtainable in an efficient, in particular cost-efficient manner. It is an object of the invention to address aforementioned problems or drawbacks of the prior art.

In this respect, the present disclosure relates to a method of producing a catalyst for synthesizing methanol from carbon dioxide and hydrogen via a catalysis, comprising a zirconium oxide support and oxide sites provided on the support including catalytic sites of zinc oxide as well as sites for capturing carbon dioxide and of an oxide of an alkaline metal and/or alkaline earth metal, the oxide sites being provided from impregnating with one or more precursor solutions for forming oxide crystals, the method comprising
providing a dry material of the zirconium oxide support, preferably a powder,
impregnating the dry material with the one or more aqueous precursor solutions, and
removing solvent from the one or more aqueous precursor solutions, preferably via heating and preferably also followed by calcining,
wherein the one or more aqueous precursor solutions contain separately or together dissolved salts of zinc and of the alkaline metal and/or alkaline earth metal. When the one or more aqueous precursor solutions contain separately the dissolved salts of zinc and of the alkaline metal and/or alkaline earth metal, the above-mentioned operations of providing a dry material, impregnating it, removing the solvent and preferably subsequent calcining are preferably performed once with
   - zirconium oxide as the material of the zirconium oxide support, and
   - one of the dissolved salts of zinc or the dissolved salt(s) of the alkaline metal and/or alkaline earth metal in the aqueous precursor solution, preferably with the dissolved salt of zinc,
and once again with
- the resulting preferably calcined product as the material of the zirconium oxide support, and
- the other one of the dissolved salts of zinc or the dissolved salt(s) of the alkaline metal and/or alkaline earth metal in the aqueous precursor solution.

More preferably the one or more aqueous precursor solutions contain together in a sense of a mixture of aqueous precursor solutions or an aqueous precursor solution combining the dissolved salts.

The present disclosure further relates to a catalyst for synthesizing methanol from carbon dioxide and hydrogen via a catalysis, comprising a zirconium oxide support and oxide sites provided on the support including catalytic sites of zinc oxide as well as sites for capturing carbon dioxide and of an oxide of an alkaline metal and/or alkaline earth metal, the oxide sites being provided from an impregnation with one or more precursor solutions for forming oxide crystals, wherein the catalyst is obtainable by the aforementioned method, preferably the method, wherein the one or more aqueous precursor solutions contain together the dissolved salts of zinc and of the alkaline metal and/or alkaline earth metal. Amounts of zinc atoms and zirconium atoms determined on this catalyst by XPS may show a ratio of an amount of zinc to an amount of zirconium of 0.4 or more, preferably 0.6 or more, more preferably 0.7 or more, most preferably 0.8 or more, preferably with an upper limit of 1.5 or less, more preferably 1.2 or less, most 1.1 or less.

The present disclosure also relates to a flow reactor for synthesizing methanol from carbon dioxide and hydrogen which is characterized and/or differs from a commonly known flow reactor for this purpose in that it comprises the catalyst according to the present disclosure.

A method for synthesizing methanol from carbon dioxide and hydrogen via a catalysis, preferably at a temperature between 180 °C and 400 °C and a pressure of less than 1.5 MPa, with the catalyst according to the present disclosure in the flow reactor according to the present disclosure comprises steps of i) establishing in the flow reactor a flow of a gas containing the carbon dioxide, like an inert atmosphere with a concentration of carbon dioxide of 20 vol% or less, for an interaction between the carbon dioxide and the catalyst, and ii) replacing in the flow reactor the flow of the gas containing the carbon dioxide by a flow of a gas containing the hydrogen and synthesizing methanol in the flow reactor with the flow of a gas containing the hydrogen. With the flow of a gas containing the carbon dioxide, carbon dioxide may also be supplied as an inert or not inert atmosphere with preferably of at most 550 ppm, particularly preferably at most 500 ppm like between 300 ppm and 500 ppm carbon dioxide.

The present disclosure also relates to a use of the catalyst according to the present disclosure for synthesizing methanol from carbon dioxide and hydrogen in the method for synthesizing methanol from carbon dioxide and hydrogen via a catalysis.

According to preferences of the above mentioned a method of producing a catalyst for synthesizing methanol from carbon dioxide and hydrogen via a catalysis the salt of zinc may be a carboxylic acid salt of zinc, the alkaline metal and/or alkaline earth metal may be selected from at least one of K, Rb, Cs, Ca, Sr and Ba, in particular so that the alkaline earth metal is Ba and the sites for capturing carbon dioxide include BaO, and/or the impregnating may be performed with an aqueous precursor solution containing together dissolved salts of zinc and of the alkaline metal and/or alkaline earth metal. The step of impregnating preferably includes an operation of dropping the aqueous solution at 0.5 to 1 ml/second onto the support heated to a temperature between 80 °C and 100 °C, more preferably alternately with an operation of grinding for obtaining a powder. In particular the step of impregnating may include impregnating the dry material with a precursor for providing catalytic sites of zinc via impregnation in which zinc is associated and/or interacts with an organic component, the precursor being preferably an aqueous solution of a carboxylic acid salt, more preferably a formate, an acetate, a propionate, an isopropionate, a butyrate, or an isobutyrate, most preferably an acetate, of zinc, preferably with a zinc concentration of 12 mol% to 25 mol%, more preferably 15 mol% to 20 mol%, and preferably with an operation of dropping the aqueous solution at 0.5 to 1 ml/min onto the support heated to a temperature between 80 °C and 100 °C, and more preferably also with performing the operation of dropping alternately with an operation of grinding for obtaining a powder, preferably with a size between 40 and 80 mesh. The step of impregnating may be followed by removing solvent from the aqueous solution with a drying step, and then preferably calcining.

An exemplary sequence of steps for processing materials into a catalyst according to the invention comprises the following steps 1 to 6:
step 1 - drying zirconium oxide (ZrO₂), preferably monoclinic ZrO₂ (m-ZrO₂), overnight at e.g. 120 °C to obtain a dry white powder as a support material,
step 2 - preparing an aqueous solution of 14 to 20 mol% of Zn²⁺(aq), preferably a solution of Zn(CH□CO□)□,
step 3 - heating the product from step 1 between 80 °C and 100 °C to obtain a dry white powder,
step 4 - slowly dropping the solution from step 2 observing a dropping speed of 0.5 and 1 ml / second, using a dropper and alternately with grinding using a pestle to obtain a homogeneous mixed powder dry white powder,
step 5 - evaporating water, e.g., using an oven evaporator at 80 °C to 100 °C and atmospheric pressure for 12 to 10 h, to obtain a dried, white powder,
step 6 - grinding and then calcining the powder from step 5 in a muffle furnace heated at e.g. 10 °C/min to a temperature between 400 °C and 600 °C and keeping this temperature for 3 to 5 hours to obtain a white powder,
   and then repeating steps 2 to 6 except that step 2 is used to prepare an aqueous solution of 5 to 9 mol% of an alkaline or earth alkaline metal salt and the product from the first execution of step 6 instead of the product from step 1 is used in step 3. Alternatively and according to a preference, the repeating steps 2 to 6 may be omitted and the aqueous solution of 14 to 20 mol% of Zn²⁺(aq) in step 2 may be one which further contains 5 to 9 mol% of an alkaline or earth alkaline metal salt so that a co-impregnation results from step 4.

A catalyst according to the invention, in particular one obtained with the above exemplary sequences of steps for processing materials into a catalyst, may be subjected to the following sequence of steps to be pretreated with hydrogen (converted into a conditioned catalyst) and used in a gas swing operation for obtaining methanol:

| **Step** | **Conditions (ranges)** |
|---|---|
| Pretreatment with H₂ | Flow rate: 5 - 50 mL/min |
| | Time: 0 - 120 min |
| | Temperature: 100 - 300 °C |
| CO₂ feed | CO₂: 0.01 - 100% |
| | Flow rate: 5 - 50 mL/min |
| | Time: 5 - 30 min |
| | Temperature: 200 - 300 °C |
| Purge with He | Flow rate: 5 - 50 mL/min |
| | Time: 0 - 7 min |
| H₂ feed | H₂: 10 - 100% |
| | Flow rate: 5 - 50 mL/min |
| | Time: 10 - 60 min |
| | Temperature: 200 - 300 °C |
| Purge with He | Flow rate: 5 - 50 mL/min |
| | Time: 0 - 7 min |

The purging with He before and/or after the hydrogen feeding step may be performed without heating or with the temperature of 200 °C to 300 °C of the hydrogen feeding step. Preferably a constant temperature is used from starting the carbon dioxide feeding step until the end of the hydrogen feeding step.

An aspect of the invention is also a flow reactor for synthesizing methanol from carbon dioxide and hydrogen comprising the catalysts described above.

According to an aspect of the invention a catalyst for synthesizing methanol from carbon dioxide and hydrogen via a catalysis, in particular the catalyst described above and of the invention, is used in a gas swing operation. Pertaining further aspects of the invention are a method with and a use of the catalyst for synthesizing methanol from carbon dioxide and hydrogen via a catalysis. This method is a method for synthesizing methanol from carbon dioxide and hydrogen via a catalysis and with gas flows over the catalyst including a gas flow including the hydrogen and a flow including carbon dioxide established before a gas flow including hydrogen. Preferably, the catalysis proceeds at a temperature between 180 °C and 400 °C, more preferably between 200 °C and 325 °C, and a pressure of less than 1.5 MPa and more preferably 1.0 MPa or less. Preferably, a selectivity to methanol in the synthesizing methanol is 80% or more, particularly preferably 90% or more, most preferably 95% or more. A methanol productivity (conversion) with the invention's catalyst and in the invention's method for synthesizing methanol is preferably 0.6 mg per g catalyst and per hour, more preferably 0.7 mg per g catalyst and per hour in an evaluation identified below in the detailed description.

A method for synthesizing methanol from carbon dioxide and hydrogen via a catalysis of the invention in the flow reactor, comprises steps of
establishing in the flow reactor a flow of a gas containing the carbon dioxide for an interaction between the carbon dioxide and the catalyst, in preferably with a concentration of 20 vol% or lower, more preferably 15 vol% or lower, particularly preferably 10 vol% or lower in an inert gas or mixture of inert gases like e.g. nitrogen or helium, and at a temperature between preferably 180 °C and 400 °C, more preferably 200 °C and 325 °C, most preferably between 250 °C and 280 °C, this step being preferably followed by purging with inert gas,
replacing in the flow reactor the flow of the gas containing the carbon dioxide by a flow of a gas containing the hydrogen and synthesizing methanol in the flow reactor with the flow of a gas containing the hydrogen, preferably in an amount between 10 weight% and 100 weight%, and at a temperature between preferably 180 °C and 400 °C, more preferably between 200 °C and 325 °C, most preferably between 250 °C and 280 °C.

The above flow containing carbon dioxide may even be one containing 400 ppm or less, preferably 300 ppm or less of the carbon dioxide in the inert gas. A lower limit of the concentration of carbon dioxide is preferably 10 ppm, more preferably 100 ppm. The catalysis proceeds at a temperature preferably between 180 °C and 400 °C, more preferably between 200 °C and 325 °C, most preferably between 250 °C and 280 °C, and a pressure of less than 1.5 MPa, more preferably 1.0 MPa or less, this step being preferably followed by purging with inert gas. Flow velocities of the gas flows (preferably from the gas flow containing carbon dioxide to a purging gas flow following the hydrogen containing gas flow) are preferably between 10 and 50 ml/min.

A particular aspect of the invention, specifically of the above-mentioned method and/or catalyst use for synthesizing methanol from carbon dioxide and hydrogen, is a process which integrates CO₂ capture and methanol production so as to benefit in particular from the catalyst's capability to interact with carbon dioxide. Then one production site and/or facility serves for the capture and conversion of carbon dioxide to produce methanol with reduced effort in terms of regeneration, equipment, energy and/or transport of gas and/or the product. In particular, under this aspect, a loop process can be formed which generates CO₂ from methanol in a conventional manner (e.g., combustion) for obtaining energy and reuses the CO₂ to store energy with the synthesis of methanol according to the invention. Accordingly, an aspect of the present disclosure is also an energy buffer.

The invention provides in particular a catalyst suitable for an excellently productive gas swing operation and e.g. at a pressure of 1 MPa or less and e.g. with a first gas flow of 10% CO₂ in an inert gas like N₂, He or Ar, preferably N₂, and further separate gas flow of H₂, e.g. 100% H₂. Thus, the invention offers cost efficiency also in terms of energy and equipment used in synthesizing or in a catalysis for synthesizing methanol. It is believed that this efficiency results from more methanol-favored surface species like formates formed during CO₂ feeding and that can be used completely for reaction with H₂ and from accessibility of catalytic sites on the invention's catalyst. The invention offers a simple synthesis procedure of catalysts and a high methanol productivity and/or selectivity at moderate effort.

### BRIEF DESCRIPTION OF THE DRAWINGS

Features, advantages, and technical and industrial significance of exemplary embodiments of the invention will be described below with reference to the accompanying drawings, in which like signs denote like elements, and wherein:
FIG. 1 shows a comparison of XRD (x-ray diffraction) patterns from catalyst preparations;
FIGS. 2A to 2D show XPS spectra from samples including catalyst preparations;
FIGS. 3A to 3C show XPS spectra from samples including catalyst preparations;
FIGS. 4A to 4C show schematically time resolved component measurement profiles in a method according to the invention.

### DETAILED DESCRIPTION OF EMBODIMENTS

### Sample Preparations

Samples were prepared with the following with materials some of which are identified with manufacturers and product numbers and with sequences of steps and products resulting from the respective steps as follows.

A series of ZnO supported on m-ZrO₂ (m-ZrO₂ = monoclinic phase ZrO₂ from Alfa Aesar #43815) catalysts were prepared by a wet impregnation method in line with above described steps 1 to 6. The m-ZrO₂ supports were dried overnight at 120 °C. The dried supports were then impregnated with an aqueous solution of Zn(CH₃COO)₂ (99.99% trace metals basis from Sigma-Aldrich #383317). Upon impregnation, the supports were heated at ca. 100 °C to constantly remove excess water, while homogeneously mixed with the solutions. Finally, the catalysts were dried overnight at 100 °C and calcined in air at 500 °C for 3 h (heating rate 10 °C/min).

Then ZnO/m-ZrO₂ catalyst was promoted with different alkali and alkaline earth metals to form alkali or alkaline earth metal oxides (AlkO), as shown in Table 1 below and to achieve preferably different 6.9mol% AlkO / 16.2 mol% ZnO / 76.9 mol% m-ZrO₂ catalysts. These catalysts were prepared via the same wet impregnation method and calcination process as described above.

In the case of the BaO-ZnO/m-ZrO₂ catalyst prepared by co-impregnation, Ba(NO₃)₂ and Zn(CH₃COO)₂ were dissolved together in water before impregnating on 3g of the m-ZrO₂ support.

**Table 1 Precursors and precursor solutions for catalyst synthesis**

| **Group** | **Metal Oxide** | **Precursor** | **Weight Precursor (g)** | **Volume Water (ml)** | **Supplier** |
|---|---|---|---|---|---|
| Alkali Metals | Li₂O | LiNO₃ | 0.2674 | 4.0 | ThermoFisher# 013405 |
| | Na₂O | NaNO₃ | 0.3275 | 3.0 | Sigma-Aldrich#1.0653 7 |
| | K₂O | KNO₃ | 0.3922 | 5.0 | ThermoFisher# 42415 |
| Alkaline Earth Metals | MgO | Mg(NO₃)₂. 6H₂O | 0.4922 | 2.0 | Sigma-Aldrich#20369 6 |
| | CaO | Ca(NO₃)₂.4 H₂O | 0.4576 | 4.0 | Sigma-Aldrich#23712 4 |
| | SrO | Sr(NO₃)₂ | 0.4099 | 4.0 | Riedel-deHaën#31633 |
| | BaO | Ba(NO₃)₂ | 0.5010 | 6.0 | Sigma-Aldrich#20275 4 |

**Table 2 Catalyst synthesis protocol via (wet) impregnation**

| **Step** | **Details** | **Product** | **Preferred range** |
|---|---|---|---|
| 1 | Dry the m-ZrO₂ supports overnight at 120 °C. | dry white powder | 100 - 150 °C. |
| 2 | Dissolve 3.03 g of Zn(CH□CO□)□, in 10 ml deionized water | Solution of Zn²⁺_{(aq)} H□CO□⁻_{(aq)}, transparent solution | Concentration 0.5 - 3 mmol/ml |
| 3 | Heat the m-ZrO₂ support at ca. 100 °C on an evaporating dish. | dry white powder | 80 - 100 °C |
| 4 | Slowly drop all Zn(CH□CO□)□ solution using a dropper alternately with grinding using a pestle or alternatively in the case of co-impregnation 0.5709 g Ba(NO₃)₂ and 0.9412 g Zn(CH₃COO)₂ in 12.0mL water on 3g of the m-ZrO₂ support as mentioned above. | Homogeneous mixed powder dry white powder | 0.5 - 1 ml / second |
| 5 | evaporate water using an oven evaporator 100 °C and at atmospheric pressure over night (around 12-14 h) | Dried, white powder | 80 - 100 °C |
| | | | 12 - 20 h |
| 6 | Calcine grinded powder from step 5 in a muffle furnace at 500 °C, ramp rate =10 °C/min for 3 h, stagnant air. | White powder | 400 - 600 °C |
| | | | 3 - 5 h |
| 7 | After obtaining the ZnO/ZrO₂ catalysts. The AlkO/ZnO/m-ZrO₂ is prepared by repeating steps 3-6 using in step 4 the precursor solutions and amounts shown in Table 1. | | |

### Evaluation and measurement

1g of a respective sample was placed in a tubular fixed-bed reactor and subjected to the following sequence of gas flows at a pressure of 1 MPa with the sequence of gas feed and purge operations being repeated to pass 10 times from start t₀ to end time t₄ (to perform 10 cycles of using the catalyst):

| **Gas flow** | **Details** | **Start time** | **End time** |
|---|---|---|---|
| Pretreatment | H₂, 30 mL/min, 90 min 300 °C | | |
| CO₂ feed | CO₂ 3 vol% in He, 40 mL/min, 20 min, 300 °C | t₀ | t₁ |
| Purge | He, 40 mL/min, 5 min, 230 °C | t₁ | t₂ |
| H₂ feed | H₂, 40 mL/min, 40 min, 230 °C | t₂ | t₃ |
| Purge | He, 40 mL/min, 5 min, no heating | t₃ | t₄ |

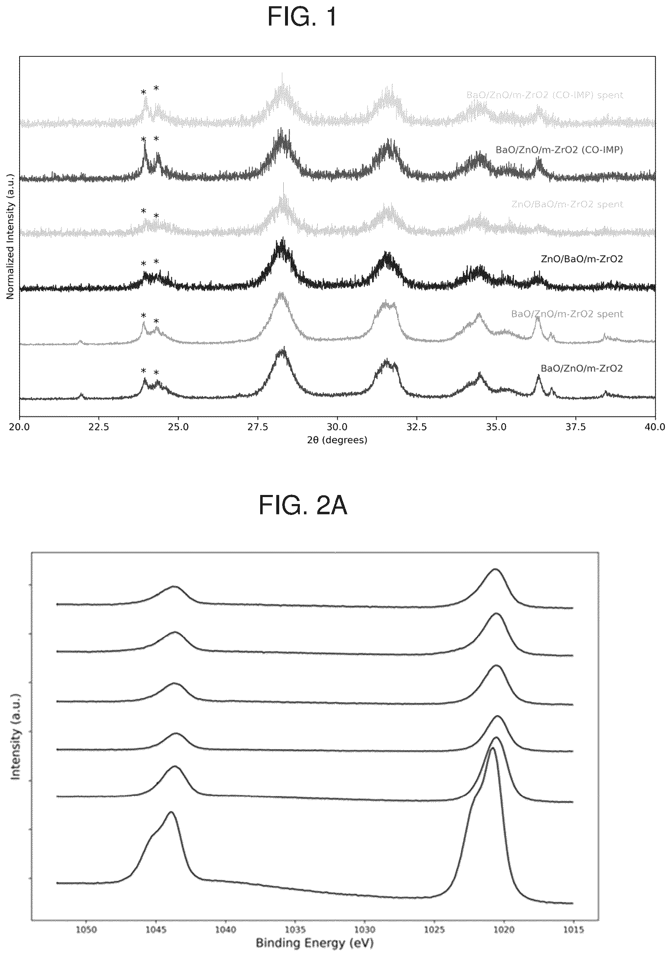

An XRD (x-ray diffraction) measurement and XPS (x-ray photoelectron spectroscopy) are commonly known techniques with commercial measurement devices. FIG. 1 shows normalized XRD spectra of samples in the following order from above to the bottom of the graph:
- BaO/ZnO/m-ZrO₂ (CO-IMP) spent - which is a sample from the co-impregnation with Zn acetate and Ba nitrate and measured after performing the 10 cycles of using the catalyst,
- BaO/ZnO/m-ZrO₂ (CO-IMP) - which is a sample from the co-impregnation with Zn acetate and Ba nitrate and measured before performing the 10 cycles of using the catalyst,
- ZnO/BaO/m-ZrO₂ spent - which is a sample from the separate impregnation, first with Ba nitrate and then with Zn acetate and measured after performing the 10 cycles of using the catalyst,
- ZnO/BaO/m-ZrO₂ - which is a sample from the separate impregnation, first with Ba nitrate and then with Zn acetate and measured before performing the 10 cycles of using the catalyst,
- BaO/ZnO/m-ZrO₂ spent - which is a sample from the separate impregnation, first with Zn acetate and then with Ba nitrate and measured after performing the 10 cycles of using the catalyst,
- BaO/ZnO/m-ZrO₂ - which is a sample from the separate impregnation, first with Zn acetate and then with Ba nitrate and measured before performing the 10 cycles of using the catalyst.

The mark * indicates peaks originating from the presence of BaCO₃, particularly prominent in case of the sample from a co-impregnation. Also, the sample ZnO/m-ZrO₂ was taken being the intermediate product in the preparation of BaO/ZnO/m-ZrO₂ before impregnating with the salt of Ba. From XRD and XPS data of these samples crystalline sizes and surface compositions were obtained according to common practice with values as follows for oxides and atoms on a catalyst or sample surface:

| sample | crystalline size from XRD (nm) | | surface composition from XPS (%) | | |
|---|---|---|---|---|---|
| | ZnO | ZrO₂ | Ba | Zn | Zr |
| ZnO/m-ZrO₂ | 43 | 13 | | 49 | 51 |
| BaO/ZnO/m-ZrO₂ | 48 | 13 | 15 | 24 | 61 |
| ZnO/BaO/m-ZrO₂ | 33 | 13 | 10 | 35 | 55 |
| BaO/ZnO/m-ZrO₂ (CO-IMP) | 34 | 13 | 9 | 42 | 50 |

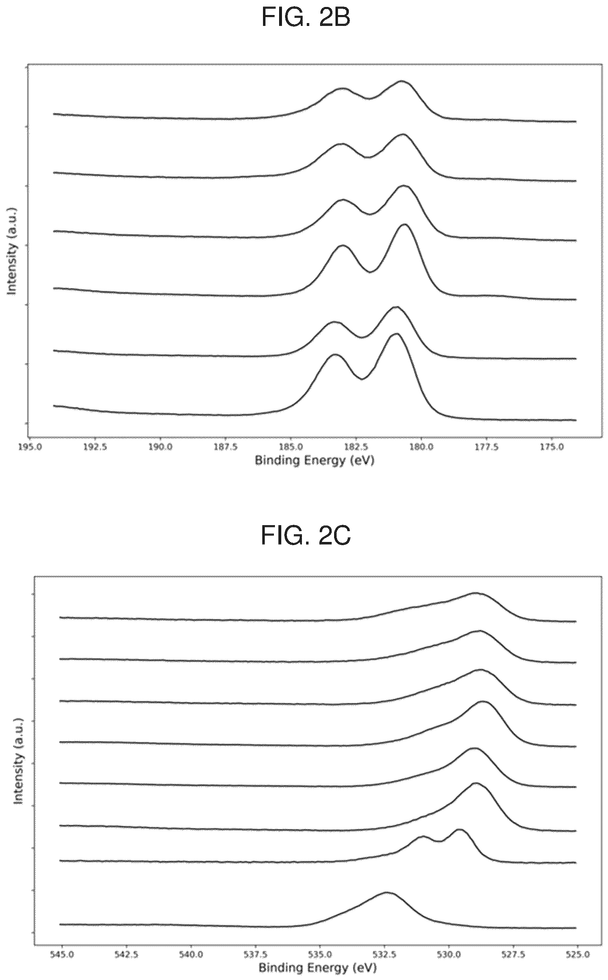

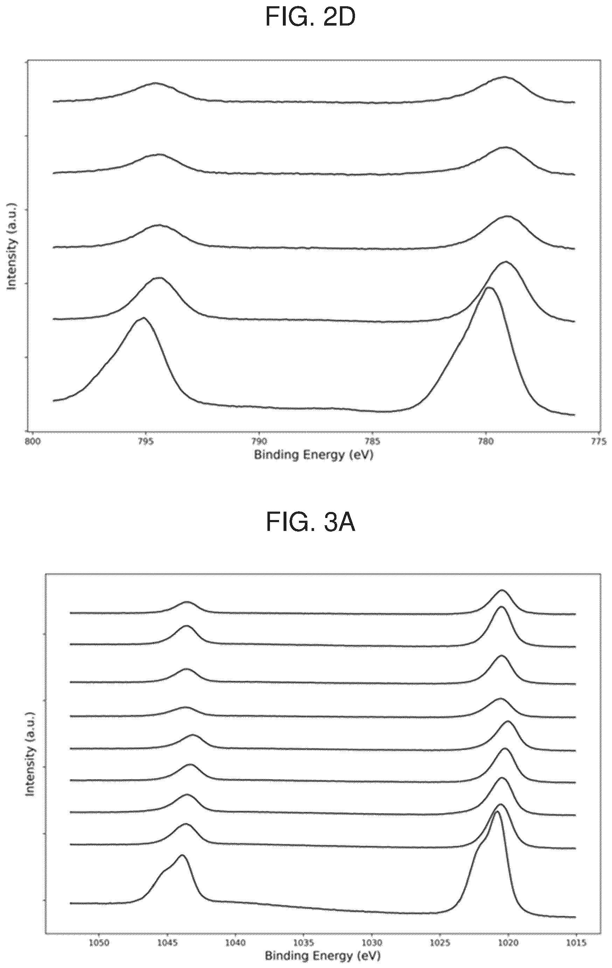

FIG. 2A shows XPS spectra measured for Zn 2p, FIG. 2B shows XPS spectra measured for Zr 3d, FIG. 2C shows XPS spectra measured for O 1s, and FIG. 2D shows XPS spectra measured for Ba 3d. Here each of FIGS. 2A to D shows spectra of the sample BaO/ZnO/m-ZrO₂ (CO-IMP) spent - which is a sample from the co-impregnation with Zn acetate and Ba nitrate and measured after performing the 10 cycles of using the catalyst on top of the respective graph and then samples (before any of using the catalyst) in the following order towards the bottom of the respective graph:
- BaO/ZnO/m-ZrO₂ (CO-IMP) - which is a sample from the co-impregnation with Zn acetate and Ba nitrate,
- ZnO/BaO/m-ZrO₂ - which is a sample from the separate impregnation, first with Ba nitrate and then with Zn acetate,
- BaO/ZnO/m-ZrO₂ - which is a sample from the separate impregnation, first with Zn acetate and then with Ba nitrate,
- ZnO/m-ZrO₂ - except in case of FIG. 2D not showing a spectrum of this sample, and which sample is taken as the intermediate product in the preparation of BaO/ZnO/m-ZrO₂ before impregnating with the salt of Ba.

In addition, in FIG. 2A the bottom most XPS spectrum is from pure ZnO, in FIG. 2B the bottom most XPS spectrum is from pure m-ZrO₂, and in FIG. 2C and D the bottom most XPS spectrum is from pure BaO and right above this spectrum FIG. 2C further shows an XPS spectrum from pure ZnO.

Accordingly, significant structural changes are obtained in catalysts depending on the variant of the method of producing them.

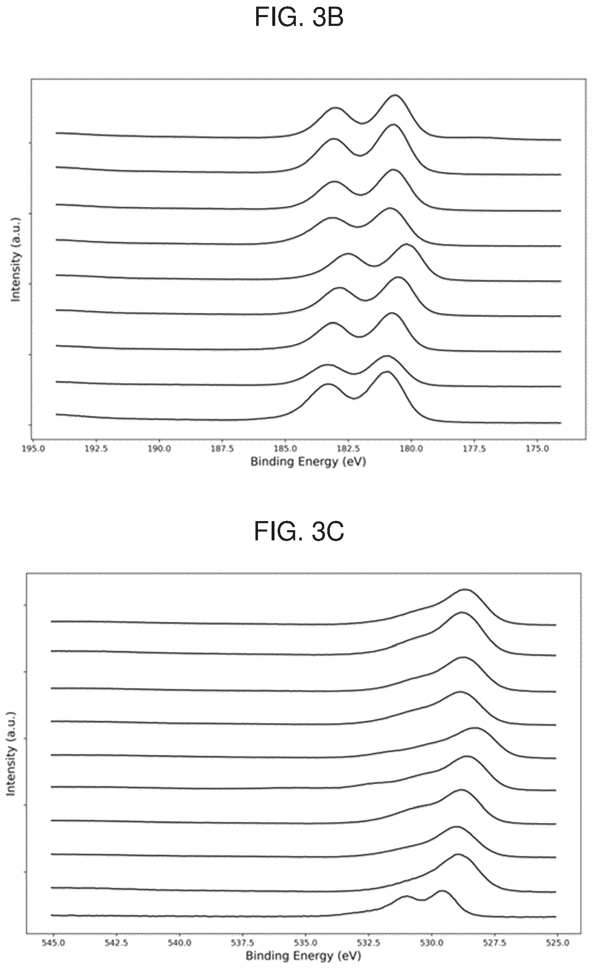

FIG. 3A shows XPS spectra measured for Zn 2p, FIG. 3B shows XPS spectra measured for Zr 3d, and FIG. 3C shows XPS spectra measured for O 1s for further samples with and without alkaline or earth alkaline oxides in the following order towards the bottom of the respective graph:
- BaO/ ZnO/m-ZrO₂ - which is a sample from the separate impregnation, first with Zn acetate and then with Ba nitrate,
- SrO/ ZnO/m-ZrO₂ - which is a sample from the separate impregnation, first with Zn acetate and then with Sr nitrate,
- CaO/ ZnO/m-ZrO₂ - which is a sample from the separate impregnation, first with Zn acetate and then with Ca nitrate,
- MgO/ ZnO/m-ZrO₂ - which is a sample from the separate impregnation, first with Zn acetate and then with Mg nitrate,
- K₂O/ ZnO/m-ZrO₂ - which is a sample from the separate impregnation, first with Zn acetate and then with K nitrate,
- Na₂O/ ZnO/m-ZrO₂ - which is a sample from the separate impregnation, first with Zn acetate and then with Na nitrate,
- Li₂O/ ZnO/m-_{ZrO2} - which is a sample from the separate impregnation, first with Zn acetate and then with Li nitrate,
- ZnO/m-ZrO₂ which is a sample from the separate impregnation with Zn acetate and no processing repetition or second impregnation with alkaline or earth alkaline metal salt solution,
- and (only) in case of FIG. 3B and C pure m-ZrO₂.

The bottom most XPS spectrum in each of FIG. 3A and 3C is from pure ZnO.

Accordingly, structural modifications are obtained over a variety of alkaline or earth alkaline oxides and not just with BaO.

The fixed-bed reactor is equipped with an in-line IR spectrometer and related analysis and monitoring system to detect chemical component concentrations in a known manner, to convert the component concentrations into molar fractions yᵢ for each respective chemical component i, and to obtain molar flows Fᵢ [mol/min] for each respective chemical component i according to the following equation: ${F}_{i} = {y}_{i} \times \frac{{v}_{T}}{22400 ml}$

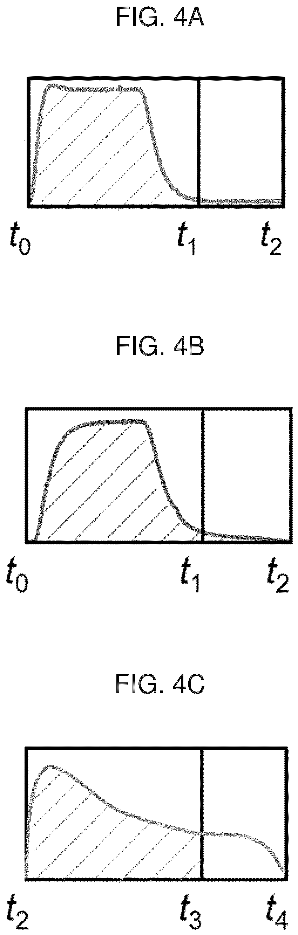

In each of FIGS. 4A to 4C the ordinate indicates correspondingly obtained molar flows Fi and the abscissa indicates a time scale on which above-mentioned start and end times t0 to t4 are indicated. Specifically in an exemplary and schematic manner
- FIG. 4A indicates a profile of a molar flow Fi, wherein the chemical component i is CO2 and the sample in the reactor is the sample ZrO₂,
- FIG. 4B indicates a profile of a molar flow Fi, wherein the chemical component i is CO2 and the sample in the reactor is the sample acetate, and
- FIG. 4C indicates a profile of a molar flow Fi, wherein the chemical component i is one of CH₄, CH₃OCH₃, CH₃OH, and CH₄, and the sample in the reactor is the sample acetate,
   and thus,
- the hatched area in FIG. 4A divided by the weight of the sample ZrO₂ used in the reactor represents the CO₂ feed amount from t0 to t2 per sample weight (A_{CO2} [mol/g (cat.)]) and in the step of establishing in the reactor a flow of a gas containing the carbon dioxide for an interaction between the carbon dioxide and the catalyst,
- the hatched area in 4B divided by the weight of the sample acetate used in the reactor represents the CO₂ feed amount from t0 to t2 per sample weight not captured in the step of establishing in the flow reactor a flow of a gas containing the carbon dioxide for an interaction between the carbon dioxide and the catalyst (sample acetate), and by subtracting this not captured amount per sample weight from A_{CO2} the captured CO₂ amount (CC_{CO2} [mol/g (cat.)]) is obtained, and
- the hatched area in FIG. 4C divided by the weight of the sample acetate used in the reactor represents an amount produced (*nᵢ*) of a chemical component i from t2 to t3 per sample weight and thus in synthesizing methanol in the flow reactor with the flow of a gas containing the hydrogen.

Same sample amounts are used in each of the measurements illustrated with FIGS. 4A to 4C. With corresponding measurements, monitoring and parameters a percentage of conversion of CO₂ (*X*_{*CO*₂}) is calculated with the following formula: ${X}_{{CO}_{2}} = \frac{\sum {n}_{i , i = {CH}_{3} OH , CO , {CH}_{4} and {CH}_{3} {OCH}_{3}}}{{CC}_{{CO}_{2}}}$

With corresponding measurements, monitoring and parameters a percentage of selectivity (Sᵢ) can be calculated from an amount (*nᵢ*) of a produced chemical component i, related to amounts of produced chemical components, like in case of the chemical component CH3OH with the following formula: ${X}_{{CO}_{2}} = \frac{\sum {n}_{i , i = {CH}_{3} OH , CO , {CH}_{4} and {CH}_{3} {OCH}_{3}}}{{CC}_{{CO}_{2}}}$

With the above-indicated measurements and performance evaluation and per gram of a respective sample used in the flow reactor amounts of captured CO₂ and of produced CH3OH (converted into mg/g (cat.)) and hourly amounts of produced CH3OH (in mg/g (cat.) h) were obtained with values as follows:

| **Sample** | **CO₂ [mg/g (cat.)]** | **CH₃OH [mg/g (cat.)]** | **CH₃OH [mg/g (cat.) h]** |
|---|---|---|---|
| ZnO/m-ZrO₂ | 2.5 | 1.023 | 0.512 |
| BaO/ZnO/m-ZrO₂ | 16.1 | 0.565 | 0.282 |
| ZnO/BaO/m-ZrO₂ | 14.9 | 0.344 | 0.172 |
| BaO-ZnO/m-ZrO₂ | 4.6 | 1.479 | 0.740 |
| Li₂O/ZnO/m-ZrO₂ | 13.2 | | |
| Na₂O/ZnO/m-ZrO₂ | 9.2 | | |
| K₂O/ZnO/m-ZrO₂ | 8.4 | | 0.127 |
| MgO/ZnO/m-ZrO₂ | 14.5 | | |
| CaO/ZnO/m-ZrO₂ | 12.3 | | 0.055 |
| SrO/ZnO/m-ZrO₂ | 14.0 | | 0.172 |

With the above-indicated measurements and performance evaluation and for respective samples used in the flow reactor percentages of conversion of CO2 and percentages of selectivity for CH3OH, CO, and CH4 were obtained with values as follows:

| **sample** | **X_{CO₂} [%]** | **S_{CH₃OH} [%]** | **S_{CO} [%]** | **S_{CH₄} [%]** |
|---|---|---|---|---|
| ZnO/m-ZrO₂ | 61.05 | 91.44 | 8.56 | 0.00 |
| BaO/ZnO/m-ZrO₂ | 5.07 | 95.28 | 4.72 | 0.00 |
| ZnO/BaO/m-ZrO₂ | 3.38 | 94.01 | 5.99 | 0.00 |
| BaO-ZnO/m-ZrO₂ | 45.10 | 97.90 | 2.10 | 0.00 |

Here the examined samples according to the invention show in particular better methanol selectivity compared to the sample ZnO/m-ZrO₂.

Although the present disclosure refers to specific exemplary embodiments, modifications may be provided to these examples without departing from the general scope of the invention as defined by the claims. In particular, individual characteristics of the different illustrated/mentioned embodiments may be combined in additional embodiments. Therefore, the description and the drawings should be considered in an illustrative rather than in a restrictive sense.

## Claims

1. A method of producing a catalyst for synthesizing methanol from carbon dioxide and hydrogen via a catalysis, comprising a zirconium oxide support and oxide sites provided on the support including catalytic sites of zinc oxide as well as sites for capturing carbon dioxide and of an oxide of an alkaline metal and/or alkaline earth metal, the oxide sites being provided from impregnating with one or more precursor solutions for forming oxide crystals, the method comprising
providing a dry material of the zirconium oxide support,
impregnating the dry material with the one or more aqueous precursor solutions, and
removing solvent from the one or more aqueous precursor solutions,
wherein the one or more aqueous precursor solutions contain separately or together dissolved salts of zinc and of the alkaline metal and/or alkaline earth metal.

2. The method according to claim 1, wherein the salt of zinc is a carboxylic acid salt of zinc.

3. The method according to claim 1 or 2, wherein the alkaline metal and/or alkaline earth metal is selected from at least one of K, Rb, Cs, Ca, Sr and Ba.

4. The method according to claim 3, wherein the alkaline earth metal is Ba and the sites for capturing carbon dioxide include BaO.

5. The method according to any of claims 1 to 4, wherein the impregnating is performed with an aqueous precursor solution containing together dissolved salts of zinc and of the alkaline metal and/or alkaline earth metal.

6. The method according to any of claims 1 to 5, wherein the dry material is a powder and the step of impregnating includes an operation of dropping the aqueous solution at 0.5 to 1 ml/second onto the support heated to a temperature between 80 °C and 100 °C.

7. The method according to claim 6, wherein the step of impregnating includes the operation of dropping alternately with an operation of grinding for obtaining a powder.

8. The method according to any of claims 1 to 7, wherein the one or more aqueous precursor solutions contain together the dissolved salts of zinc and of the alkaline metal and/or alkaline earth metal.

9. A catalyst for synthesizing methanol from carbon dioxide and hydrogen via a catalysis, comprising a zirconium oxide support and oxide sites provided on the support including catalytic sites of zinc oxide as well as sites for capturing carbon dioxide and of an oxide of an alkaline metal and/or alkaline earth metal, the oxide sites being provided from an impregnation with one or more precursor solutions for forming oxide crystals, wherein the catalyst is obtainable by the method according to any of claims 1 to 8.

10. The catalyst according to claim 9, wherein the catalyst is obtainable by the method according to claim 8.

11. A flow reactor for synthesizing methanol from carbon dioxide and hydrogen comprising the catalyst according to claim 9 or 10.

12. A method for synthesizing methanol from carbon dioxide and hydrogen via a catalysis with the catalyst according to claim 9 or 10 in the flow reactor according to claim 11, comprising steps of
establishing in the flow reactor a flow of a gas containing the carbon dioxide for an interaction between the carbon dioxide and the catalyst, and
replacing in the flow reactor the flow of the gas containing the carbon dioxide by a flow of a gas containing the hydrogen and synthesizing methanol in the flow reactor with the flow of a gas containing the hydrogen.

13. The method according to claim 12, wherein the catalysis proceeds at a temperature between 180 °C and 400 °C and a pressure of less than 1.5 MPa.

14. The method according to claim 12 or 13, wherein the gas flow including carbon dioxide constitutes an inert atmosphere with a concentration of carbon dioxide of 20 vol% or less.

15. A use of the catalyst according to claims 9 or 10 as a catalyst for synthesizing methanol from carbon dioxide and hydrogen in the method according to any of claims 12 to 14.
